# EUROPEAN PATENT APPLICATION

(11) **EP 4 005 640 A1**
(43) Date of publication of application: **01.06.2022**
(21) Application number: 20846916.3
(22) Date of filing: 15.07.2020
(51) Int. Cl.: A61Q 1/00, A61K 8/86

(54) **COSMETIC AND METHOD FOR PRODUCING COSMETIC**

(30) Priority: 31.07.2019 JP 2019140666
(71) Applicant: Miyoshi Kasei, Inc., Tokyo 102-0073 (JP)
(72) Inventor: HASEGAWA, Yukio, Tokyo 102-0073 (JP)
(74) Representative: Santarelli
(86) International application number: PCT/JP2020/027547
(87) International publication number: WO 2021/020135

(57) **Abstract**

To improve a fresh sense of use, an excellent feeling of use of hydrophobic powder, color development, transparency, ultraviolet shielding ability, and the like. In production of a cosmetic containing, respectively, 1 wt% or more of water and hydrophobic powder, (A) hydrophilic nonionic surfactant as monoether compound of branched higher alcohol having 12 to 20 carbons and polyoxyethylene group and (B) water are mixed, and then mixed with (C) hydrophobic powder.

## Description

### TECHNICAL FIELD

### [CROSS-REFERENCE TO RELATED APPLICATION]

The present invention is based on the priority of Japanese patent application No. 2019-140666 (filed on July 31, 2019), the disclosure thereof is incorporated herein in its entirety by reference thereto. The present invention relates to a producing method of cosmetic containing, respectively, 1 wt% or more of water and hydrophobic powder, in which the method comprises a step of mixing a component (A) of hydrophilic nonionic surfactant as monoether compound of branched higher alcohol having 12 to 20 carbons and polyoxyethylene group and a component (B) of water, and subsequently mixing with a component (C) of hydrophobic powder. Particularly, the present invention relates to a producing method of cosmetics, respectively, containing 0.1 wt% of a component (A) of hydrophilic nonionic surfactant as monoether compound of branched higher alcohol having 14 to 20 carbons and 5 to 20 moles of ethylene oxide, a component (B) of water, and a component (C) of hydrophobic powder. According to the producing method of the present invention, the cosmetics may contain components having conflicting properties as water and hydrophobic powder. Therefore, the producing method of the present invention may provide cosmetics having flesh sense of use, excellent sense of use of the hydrophobic powder, make-up effect of uniform finish, and make-up durability.

### BACKGROUND ART

When cosmetics contain water, it is expected that the cosmetics provide flesh sense of use, moisture feeling, and flesh feeling upon water is evaporated. In addition, the water is an essential component in a living body and having extremely high safety, thus it is a component to be desired to be contained as much as possible. On the other hand, the hydrophobic powder is a powder that repels water, and even if water and the hydrophobic powder are mixed, not only they are not dispersed, but also the hydrophobic powder particles are aggregated due to the hydrophobic interaction. In recent years, various technologies for blending hydrophobic powder with aqueous cosmetics have been disclosed (Patent Literatures (PTLs) 1 to 3). Further, a technology for blending water with a powder foundation is also disclosed (Patent Literature 4).

### CITATION LIST

### PATENT LITERATURE

[PTL 1] WO2016/2752A
[PTL 2] JP2015-78243A
[PTL 3] JP2015-203026A
[PTL 4] JP3993833B

### SUMMARY

### TECHNICAL PROBLEM

However, there are problems to be solved that are not sufficient to obtain a cosmetic having a fresh sense of use, an excellent feeling of use of hydrophobic powder, a make-up effect that can obtain a uniform finish, and a cosmetic having make-up durability.

In particular, there is a problem to be solved in order to obtain a cosmetic having a uniform finish as a cosmetic effect while exhibiting color development, a transparent texture, and an ultraviolet shielding ability.

### SOLUTION TO PROBLEM

The present invention has been made in view of the above-mentioned problems. It was found that the cosmetics produced by a cosmetic producing method comprising a step of mixing a component (A) of a hydrophilic nonionic surfactant as a monoether compound of a branched higher alcohol having 12 to 20 carbons and a polyoxyethylene group and a component (B) of water, subsequently mixing a component (C) of hydrophobic powder may further improve a fresh sense of use, an excellent feeling of use of the hydrophobic powder, color development, transparency, ultraviolet shielding ability and the like. In particular, it was found that a cosmetic containing, respectively, 0.1 wt% or more of a component (A) of hydrophilic nonionic surfactant as a monoether compound of branched higher alcohol having 14 to 20 carbons and 5 to 20 moles of ethylene oxide, a component (B) of water, and a component (C) of hydrophobic powder, which is produced by a cosmetic producing method comprising a step of mixing the component (A) and the component (B) and subsequently mixing and dispersing the component (C) or a cosmetic producing method comprising a step of mixing or contacting the component (A) and the component (C), subsequently adding them to the component (B) and mixing and dispersing them, may further improve a fresh sense of use, an excellent feeling of use of the hydrophobic powder, color development, transparent texture, ultraviolet shielding ability and the like.

That is, according to a first aspect of the present invention, there is provided a producing method of cosmetic containing, respectively, 0.1 wt% or more of water and hydrophobic powder, in which the method comprises a step of mixing a component (A) of a hydrophilic nonionic surfactant as a monoether compound of a branched higher alcohol having 12 to 20 carbons and a polyoxyethylene group and a component (B) of water, and subsequently mixing a component (C) of a hydrophobic powder.

In particular, there is provided a producing method of cosmetics containing, respectively, 0.1 wt% or more of a component (A) of a hydrophilic nonionic surfactant as a monoether compound of a branched higher alcohol having 12 to 20 carbons and 5 to 20 moles of ethylene oxide, a component (B) of water, and a component (C) of hydrophobic powder, in which the method comprises a step of mixing the component (A) and the component (B), and subsequently mixing and dispersing the component (C), or the method comprises a step of mixing or contacting the component (A) and the component (C) and subsequently adding them to the component (B) and mixing and dispersing them.

In the above first aspect, it is preferable that the component (B) water contains less than 50 wt% of polyol.

In particular, it is preferable that the component (B) contains 10 wt% or less of a component capable of being mixed or solubilized in the component (B), that is other than ethyl alcohol or polyhydric alcohol. Further, it is preferable that the component (B) contains 50 wt% or less of a polyhydric alcohol. Further, it is preferable that the component (B) contains 10 wt% or less of a component capable of being mixed or solubilized in the component (B), that is other than ethyl alcohol or polyhydric alcohol, and the component (B) further contains 50 wt% or less of polyhydric alcohol.

In the above first aspect, it is preferable that the component of the component (C) is an organic powder.

In the above first aspect, it is preferable that the component of the component (C) is a hydrophobic inorganic powder.

In the above first aspect, it is preferable that the component of the component (A) is a monoether compound of a branched higher alcohol having 14 to 20 carbons and a polyoxyethylene group.

In particular, it is preferable that the component of (A) is a monoether compound of a branched higher alcohol having 16 to 20 carbons and 10 to 20 moles of ethylene oxide.

According to a second aspect of the present invention, there is provided a cosmetic produced by the producing methods of the first aspect.

### ADVANTAGEOUS EFFECTS OF INVENTION

According to the present invention, in the production of cosmetics containing, respectively, 1 wt% or more of water and hydrophobic powder, component (A) of hydrophilic nonionic surfactant as monoether compound of branched higher alcohol having 12 to 20 carbons and polyoxyethylene group is mixed with component (B) of water, and subsequently mixed with component (C) of hydrophobic powder. The cosmetic using the cosmetic producing method including this step can further improve fresh sense of use, excellent sense of use of the hydrophobic powder, color development, transparency, and ultraviolet shielding ability and the like.

Particularly, in cosmetics containing, respectively, 0.1 wt% or more of component (A) of hydrophilic nonionic surfactant as ether compound of branched higher alcohol having 14 to 20 carbons and 5 to 20 moles of ethylene oxide, component (B) of water, and component (C) of hydrophobic powder, the cosmetics provided using a cosmetic producing method comprising a step of mixing the component (A) and the component (B), and subsequently mixing and dispersing the component (C), or a cosmetic producing method comprising a step of mixing or contacting the component (A) and the component (C), and subsequently adding the resulting mixture to the component (B) followed by mixing and dispersing can further improve fresh sense of use, excellent sense of use of the hydrophobic powder, color development, transparency, and ultraviolet shielding ability and the like.

### MODES

The present invention is explained in detail as follows.

(Component (A) of hydrophilic nonionic surfactant as monoether compound of branched higher alcohol having 12 to 20 carbons and polyoxyethylene group, or hydrophilic nonionic surfactant as monoether compound of branched higher alcohol having 14 to 20 carbons and 5 to 20 moles of ethylene oxide)

A specific hydrophilic surfactant used in the present invention refers to a nonionic surfactant having a hydrophobic moiety based on a branched higher alcohol and a hydrophilic moiety based on a polyoxyethylene group. Particularly, preferable is an ether compound of a branched higher alcohol has 12 to 20 carbons and a polyoxyethylene (hereinafter, referred to as POE).

In addition, preferable is an ether compound of a branched higher alcohol having 16 to 20 carbons and 5 to 20 moles of ethylene oxide (hereinafter, the ethylene oxide polymer is referred to as POE and referred to as POE (5) when the number of moles added is 5). More preferable is a monoether compound of branched higher alcohol having 16 to 20 carbons and 10 to 20 moles of ethylene oxide.

If the number of carbons is less than 14, the dispersibility of the hydrophobic powder in water or aqueous phase is worsened, and if the number of carbons is larger than 20, the affinity of the nonionic surfactant with water is worsened, resulting in that dispersibility of hydrophobic powder in water or aqueous phase is also worsened. If the number of moles added in the ethylene oxide is smaller than 5 and larger than 20, the dispersibility of the hydrophobic powder in water or an aqueous phase tends to be inferior. Further, if the carbon chain is a saturated or unsaturated straight type, the dispersibility of the hydrophobic powder in water or an aqueous phase is significantly worsened. The specific hydrophilic nonionic surfactant of the present invention may easily and uniformly mix and disperse hydrophobic powder in water or an aqueous phase, thus may develop conventional cosmetics by added during production process of the cosmetics.

The hydrophilicity of the hydrophilic nonionic surfactant of the present invention is preferably in a range of 8 to 16 in terms of HLB. HLB referred in the present invention is defined by the following formula. HLB = (Molecular weight of hydrophilic moiety (POE) in surfactant / Molecular weight of surfactant) × 20

The hydrophilic nonionic surfactant in the present invention is an essential component for easily and stably mixing water and hydrophobic powder to obtain a mixture.

The hydrophilic nonionic surfactants which are generally available, but exert the effects of the present invention are exemplified by Nonion IS-205 (POE-5 isostearyl ether), Nonion IS-210 (POE-10 isostearyl ether), Nonion IS-215 (POE-15 isostearyl ether), Nonion IS-220 (POE-20 isostearyl ether), Nonion OD-220 (POE-20 octyldodecyl ether) of NOF Corporation, and the like. Also exemplified by EMALEX1605 (POE-5 hexyldecyl ether), EMALEX1610 (POE-10 hexyldecyl ether), EMALEX1615 (POE-15 hexyldecyl ether), EMALEX1805 (POE-5 isostearyl ether), EMALEX1810 (POE-10 isostearyl ether), EMALEX1815 (POE-15 isostearyl ether), EMALEX OD-20 (POE-20 octyldodecanol ether) of Nihon Emulsion Co., Ltd., and the like.

### (Component (B) of water)

Water of the present invention refers to ion exchanged water, distilled water, or the like, which may be used for cosmetics. In the present invention, a system containing a component of alcohol dissolvable in at least water, that is other than water as a main component, may be used. Aqueous solvent contains at least alcohols while containing water as a main component. The component as the alcohols is exemplified by ethanol, benzyl alcohol, phenoxyethanol, propylene glycol, dipropylene glycol, 1,3 butylene glycol, polyethylene glycol, glycerin, diglycerine, polyglycerol, hexyl glycerin, cyclohexyl glycerin, trimethylolpropane, xylitol, erythritol, trehalose, sorbitol, and the like. One or more components may be contained therein. Here, there is also a case where the aqueous solvent is referred to simply "water" in the present application.

As components other than ethyl alcohol or polyhydric alcohol that are mixed or solubilized in water, that is, components in the aqueous phase are exemplified by ultraviolet absorbers, water-soluble polymers, organic thickeners, inorganic thickeners, swelling agents, moisturizers, emollients, antibacterial agents, preservatives, fragrances, salts, antioxidants, pH regulators, chelating agents, refreshing agents, anti-inflammatory agents, skin-beautifying ingredients, skin astringents, vitamins, amino acids, and the like. These components are preferably contained in the component (B) of water in an amount of 10 wt% or less. Mixing refers to that water and other components are dissolved or mixed, and the other components may be in the form of a liquid or a solid at room temperature. Solubilization(dissolving) refers to a state in which a component that is originally immiscible in water is transparently dissolved in water due to the presence of a hydrophilic surfactant. Further, polyhydric alcohols contained in the aqueous phase in an amount of 50 wt% or less in the component (B) are exemplified by pentandiol, hexanediol, propylene glycol, dipropylene glycol, 1,3 butylene glycol, polyethylene glycol, glycerin and diglycerin , polyglycerin, hexylglycerin, cyclohexylglycerin, trimethylolpropane, xylitol, erythritol, trehalose, sorbitol and the like. One or more of these components can be contained therein. The polyhydric alcohol is preferably one or more selected from dipropylene glycol, 1,3 butylene glycol, and glycerin.

The mixing ratio of water and alcohol in the aqueous solvent is water / alcohol = 100/0 to 50/50 (wt%). If the mixing ratio of the alcohol is more than 50 wt%, the hydrophobic powder of the present invention tends to have poor dispersibility in an aqueous solvent.

In the present invention, an aqueous phase is also listed, which contains a component other than ethyl alcohol or polyhydric alcohol that is mixed or dissolved in the component (B) in an amount of 10 wt% or less, and further contains polyhydric alcohols in the component (B) in an amount of 50 wt% or less.

If the mixing ratio of the polyhydric alcohol in the component (B) is more than 50 wt%, the hydrophobic powder of the present invention tends to have poor dispersibility in the aqueous phase, and resulting in that the characteristics of the hydrophobic powder cannot be exhibited in cosmetics.

Other components of the aqueous solvent include water-soluble ultraviolet absorbers, water-soluble polymers, water-soluble thickeners, water-soluble swelling agents, moisturizers, antibacterial preservatives, fragrances, salts, antioxidants, pH adjusters, chelating agents, refreshing agents, anti-inflammatory agents, skin-beautifying ingredients, skin astringents, vitamins, amino acids, and the like.

### (Component (C) of hydrophobic powder)

The hydrophobic powder used in the present invention is an organic powder having hydrophobicity or a hydrophobic inorganic powder coated with an organic surface treatment agent. The organic powder is exemplified by (meth) alkyl acrylate powder, polyethylene powder, polypropylene powder, polystyrene powder, polyethylene terephthalate powder, polytetrafluoroethylene powder, polyurethane powder, polylactic acid powder, nylon 12 powder, organic resin powders such as octanoyl lysine, lauroyl lysine, etc., silicone powders such as silicone elastomer powder, polymethylsilsesquioxane, etc., and metal soap powders such as calcium stearate, zinc stearate, and magnesium stearate, etc. The average particle size is within a range of 0.1 to 500 µm. The particle shape may be spherical, hemispherical, flaky, needle-shaped, amorphous, or any other shape.

The organic powders may also include carbon black, hydrophobicized cellulose powder, starch powder, etc.

The hydrophobic organic powders as silicone powders in the present invention which is generally available are exemplified by, for example, as trade names, KSP-100, KSP-101, KSP-102, KSP-105, KSP-300, KSP-411, KSP-441, KMP-590, and KMP-591 from Shin-Etsu Chemical Co., Ltd. Polyethylene terephthalate powders are exemplified by, as trade names, Snow Leaf P and Snow Leaf PF, polyurethane powders are exemplified by, as trade names, DAIMIC BEAZ from Dainichi Seika Co., Ltd., metal soap powders are exemplified by, as trade names, Calcium Stearate S and Aulabrite NC, Aulabrite NM, Zincstearate S of NOF Corporation, and amino acid powders are exemplified by, as trade names, Amihope LL as lauroyl lysine, Amihope OL as caproyl lysine.

Further, carbon black is exemplified by, as trade names, C47-2222 SunCROMA D&C Black 2.

The organic surface treatment agent for the hydrophobic inorganic powder coated with the organic surface treatment agent in the present invention is exemplified by one or more compounds selected from silicone compounds, alkylsilanes, alkyltitanates, polyethylenes, acylated amino acids, fatty acids, lecithin, ester oils, fructo-oligosaccharides, acrylic polymers and urethane polymers.

In addition, the organic surface treatment agent may include alkyl phosphoric acid and the like.

As the silicone compound used in the present invention, methylhydrogenpolysiloxane (Shinetsu Chemical Industry Co., Ltd .: KF99P, KF9901, X-24-9171, X-24-9221, etc.), dimethiconol, one-end alkoxysilyldimethylpolysiloxane, trimethylsiloxysilicic acid, cyclic methylhydrogen silicones such as tetrahydrotetramethylcyclotetrasiloxane, acrylic silicones, silicone acrylics, amino-modified silicones, carboxy-modified silicones, phosphate-modified silicones and the like can also be used. In addition, also used are, as commercially available from Shin-Etsu Chemical Co., Ltd., KF-9908 (triethoxysilylethyl polydimethylsiloxyethyl dimethicone), KF-9909 (triethoxysilylethyl polydimethylsiloxyethyl hexyl dimethicone) and the like.

The alkylsilane used in the present invention is exemplified by alkylalcosixylan. The length of the alkyl chain is exemplified by 1 to 18 carbons, and concretely by methyltriethoxysilane, octyltriethoxysilane, octadecyltriethoxysilane, and aminopropyltriethoxysilane.

In addition, the alkylsilane is exemplified by hexyltriethoxysilane.

The organic titanate used in the present invention has a Ti (OR1) 4 structure as a basic skeleton, and R1 is independent of each other and is an alkyl group or an organic carbonyl group. Commercially available one is exemplified by isopropyltriisostearoyl titanate (Plenact TTS; Ajinomoto Fine Techno Co., Ltd.).

The polyolefin used in the present invention is exemplified by polyethylene, polypropylene and the like, which are polyolefin resins having at least one carboxyl group in the molecule. For example, it is exemplified by low molecular weight polyethylene having a molecular weight of 500 to 40,000 and a melting point of 40 °C or higher, which is a known compound described in JPSho63-179972A, polyethylene oxide obtained by oxidizing polypropylene, maleic polyethylene, polypropylene oxide, etc.

The hydrogenated lecithin used in the present invention is natural lecithin extracted from egg yolk, soybean, corn, rapeseed, sunflower, etc. and hydrogenated synthetic lecithin, which has an iodine value of 15 or less and a glyceride having a phosphate group. The salt form thereof is exemplified by water-insoluble hydrogenated lecithin metal salts such as Al, Mg, Ca, Zn, Zr and Ti.

Hydrogenated lecithin may be a preferable example of lecithin used in the present invention.

The acylated amino acid used in the present invention is an acylated compound of saturated fatty acids having 12 or more and 18 or less carbons and an amino acid selected from aspartic acid, glutamic acid, alanine, glycine, sarcosin, proline, hydroxyproline, a total hydrolyzate of a plant-derived peptide, such as wheat, beans or the like, silk peptides, animal-derived peptides, etc. A carboxyl group of the amino acids may be a free form, or a salt of K, Na, Fe, Zn, Ca, Mg, Al, Zr, Ti or the like. Concretely, it is exemplified by Amisoft CS-11, CS-22, MS-11, HS-11P, HS-21P, etc., which are commercially available from Ajinomoto Co., Inc., and Soipon SLP, Soipon SCA, Alanon AMP, which are commercially available from Kawaken Fine Chemical Co., Ltd., SEPILIFT DPHP and the like, which are commercially available from SEPPIC in France, and sarcosinate MN and the like, which are commercially available from Nikko Chemical Co., Ltd. These acylated amino acids may be in the form of a composition with a fatty acid. The acylated lipoamino acid composition is exemplified by SEPIFEEL ONE (a composition comprising four components of palmitoyl proline, palmitoyl sarcosine, palmitoyl glutamic acid, and palmitic acid) commercially available from SEPPIC.

The acidic ester oil used in the present invention comprises an ester compound having a total carbon number of 16 or more, which may be obtained by reacting one or more alcohols having 1 to 36 carbons with one or more carboxylic acids having 1 to 36 carbons. A compound having an acid value of 15 or more is suitable. Known compounds are disclosed in JP2004-51945A, and concretely exemplified by Saracos MIS (isostearyl sebacate), Saracos MOD (octyldodecanol azelaic acid), and Saracos 1A (Octyldodecanol adipate), Saracos HD (octyldodecanol dimerate) and the like, which are commercially available from Nisshin Oillio Group.

The fatty acid used in the present invention is a linear or branched saturated or unsaturated fatty acid having 12 to 22 carbons, and exemplified by, for example, lauric acid, myristic acid, palmitic acid, stearic acid, oleic acid, linole. Fatty acids such as acid, palmitoleic acid, behenic acid, lignoseric acid, 2-ethylhexanoic acid, isotridecanic acid, isomyristic acid, isopalmitic acid, isostearic acid, isobehenic acid, or metal salts thereof, such as. Ca, Mg, Zn, Zr, Al, Ti and the like.

The dextrin fatty acid ester and fructooligosaccharide ester used in the present invention may be selected from an ester composed of dextrin or fructooligosaccharide and a fatty acid or a derivative thereof. Concretelly, it is exemplified by, for example, Leopard KL, Leopard MKL, Leopard TT, Leopard KE, Leopard TL, Leopard ISK and the like, which are known compounds described in JPHei5-3844A and JP2002-188024A and are commercially available from Chiba Flour Milling Co., Ltd.

The acrylic polymer used in the present invention includes a copolymer of one or more monomers composed of acrylic acid or methacrylic acid and alkyl acrylate, and, is exemplified by, as the INCI name, (Acrylate / alkyl acrylate (C10-30)) crosspolymer, (Acrylate / Behenez Methacrylic Acid-25) Copolymer, (Acrylate / Steareth Methacrylate-20) Cross Polymer and the like.

The polyurethane polymer used in the present invention is exemplified by, as the INCI name, (PEG-240 / decyltetradeceth-20 / HDI) copolymer (Adecanol GT-700: ADEKA), and bisstearyl PEG / PPG-8 / 6 (SMDI / PEG-400) copolymer (Akpek HU C2002: Sumitomo Seika Co., Ltd.) and the like, which are a polymer having a hydrophilic moiety of a polyurethane skeleton and a hydrophobic moiety in the molecule. Further, the alkyl phosphoric acid used in the present invention is exemplified by a monoester, diester or triester of a long-chain alkyl alcohol and phosphoric acid and exemplified by lauryl phosphoric acid, cetyl phosphoric acid and C20-22 phosphoric acid. Alternatively, K- or Na- salts of these alkyl phosphoric acids may be used.

The inorganic powder used in the present invention is not particularly limited as long as it is an inorganic powder usually used for cosmetics. For example, it is exemplified by sericite, natural mica, calcined mica, synthetic mica, synthetic sericite, alumina, mica, talc, kaolin, bentonite, smectite, calcium carbonate, magnesium carbonate, magnesium silicate, aluminum silicate, calcium phosphate, silicic acid anhydride, magnesium oxide, barium sulfate, magnesium aluminometasilicate, iron oxide, chromium oxide, titanium oxide, zinc oxide, cerium oxide, aluminum oxide, magnesium oxide, dark blue, ultramarine, calcium carbonate, magnesium carbonate, calcium phosphate, aluminum hydroxide, magnesium sulfate, silicic acid, magnesium aluminum silicate, calcium silicate, barium silicate, strontium silicate, silicon carbide, metal tungstate, magnesium aluminate, magnesium aluminometasilicate, chlorhydroxyaluminum, clay, zeolite, hydroxyapatite, ceramic powder, aluminum nitride, silicone carbite, cobalt titanate, lithium cobalt titanate, cobalt aluminate, inorganic blue pigment, low-order titanium oxide, fine particle titanium oxide, butterfly-like barium sulfate, petal-like zinc oxide, hexagonal plate zinc oxide, tetrapot-like zinc oxide, fine particle zinc oxide, titanium oxide-coated mica, titanium oxide-coated mica, titanium oxide-coated silica, titanium oxide-coated synthetic mica, titanium oxide-coated talc, fish scale foil, titanium oxide-coated colored mica, titanium oxide coated borosilicic acid (sodium / calcium), titanium oxide-coated lauric acid (calcium / aluminum), bengala-coated mica, bengala-coated mica titanium, bengala-black iron oxide-coated mica titanium, carmine-coated mica titanium, carmine-conjo-coated mica titanium, mango violet, cobalt violet, glass fiber, alumina fiber, and the like.

The production method for obtaining the hydrophobic inorganic powder referred to in the present invention is not particularly limited, and it may be produced by mixing the organic surface treatment and the inorganic powder. The mixing method is not particularly limited, such as a dry method, a wet method or the like, and a mixer capable of uniform processing may be adopted. For example, it is exemplified by a Henschel mixer (Henschel mixer), a ribbon blender, a kneader, an extruder, a disper mixer, a homomixer, a bead mill and the like. After mixing, the powder can be obtained by drying with a hot air dryer, a spray dryer, a flash dryer, a conical dryer or the like.

The flash dryer can also be referred to as a flash jet dryer.

The hydrophobic powder of the present invention is a powder having hydrophobicity. As an evaluation method, putting 100 cc of purified water in a 200 cc glass beaker, dropping 0.2 g of the powder collected in a spartel from a height of 2 cm above the water surface onto the water surface. Then stirring 50 times using a spartel twice a second and letting stand for 30 seconds. When observing water, it is preferable that the powder particles float without migrating to the aqueous layer.

In addition, putting 100 cc of purified water in a 200 cc glass beaker, taking 0.2 g of powder in a spatula, dropping 0.2 g of the powder collected in a spartel from a height of 2 cm above the water surface onto the water surface. Then stirring 50 times using a spartel twice a second and letting stand for 30 seconds. It is also preferable that the powder particles float without migrating to the aqueous layer.

The method for producing the cosmetic of the present invention will be described below. The present invention is a method for producing a cosmetic containing 1 wt% of water and hydrophobic powder, in which the method comprises a step of mixing a component (A) of hydrophilic nonionic surfactant as an ether compound of a branched higher alcohol having 12 to 20 carbons and a polyoxyethylene group and a component (B) of water, subsequently mixing with a component (C) of hydrophobic powder.

In particular, the present invention is a method for producing a cosmetic containing, respectively, 0.1 wt% or more of component (A) of hydrophilic nonionic surfactant as a monoether compound of a branched higher alcohol having 16 to 20 carbons and 5 to 20 moles of ethylene oxide, component (B) of water, and component (C) of hydrophobic powder, in which the method comprises a step of mixing the component (A) and the component (B) and then mixing and dispersing the component (C) or a step of mixing or contacting the component (A) and the component (C) and then adding them to the component (B) to mix and disperse.

In the production process of the cosmetic of the present invention, first, the component (A) of hydrophilic nonionic surfactant and the component (B) of water are mixed. The ratio of the component (A) to the component (B) is not particularly limited and varies depending on the function and effect of the cosmetic to be produced. The method for mixing the component (A) and the component (B) is not particularly limited, and a known mixer can be used. For example, a mixer such as a propeller stirrer, a disper mixer, a homomixer, a high-pressure homomixer, a kneader, a heschel mixer, a V-type mixer, a roll mill, a bead mill, and an extruder can be used.

If the water or aqueous phase of the component (B) is heated to 50 °C or higher in order to improve the miscibility of the component (A) with water, the wettability of the hydrophobic powder tends to be improved and the dispersion time tends to be shortened.

The component (B) may contain, as another component, within a range as long as the effect of the present invention is not impaired when the component (A) and the component (B) are mixed, a component other than ethyl alcohol or polyhydric alcohol at an amount of 10 wt% or less, which is miscible or solubilized in the component (B). Further, the component (B) may contain a polyhydric alcohol in an amount of 50 wt% or less. Further, a component other than ethyl alcohol or polyhydric alcohol to be miscible or solubilized in the component (B) and the polyhydric alcohol may be simultaneously contained in an amount of 50 wt% or less. Since polyhydric alcohol lowers the surface tension of water, the affinity of the hydrophobic powder for the aqueous phase improves, that is, the hydrophobic powder easily enters the aqueous phase, but the more the amount of polyhydric alcohol, dispersibility of the hydrophobic powder in the aqueous phase tends to be worsened.

In the cosmetic producing process of the present invention, there is no particular limitation on the method of mixing or contacting the component (A) and the component (C), and a known mixer can be used. For example, a mixer such as a propeller stirrer, a kneader, a Henschel mixer, a V-type mixer, a roll mill, a bead mill, or an extruder can be used. After the component (A) and the component (C) are mixed or brought into contact with each other, they may be added to the component (B) and mixed / dispersed by a known mixer. at this stage, the component (B) may contain, within a range as long as the effect of the present invention is not impaired, as another component, a component other than ethyl alcohol or polyhydric alcohol at 10 wt% or less, which is miscible or solubilized in the component (B). Further, the component (B) may contain a polyhydric alcohol in an amount of 50 wt% or less. Further, the component (B) may simultaneously contain a component other than ethyl alcohol or polyhydric alcohol to be miscible or solubilized in the component (B) and the polyhydric alcohol in an amount of 50 wt% or less.

Optional component other than the ethyl alcohol or polyhydric alcohol that can be blended in the water of the component (B) is not particularly limited, but exemplified by, for example, a thickener, a moisturizer, an oil agent, an ultraviolet absorber, and a pH adjuster Neutralizing agents, antioxidants, preservatives, chelating agents, emollients, plant extracts, fragrances, pigments, various agents and the like other than the above.

Next, the component (C) of hydrophobic powder is mixed with the mixture of the component (A) of hydrophilic nonionic surfactant and the component (B) of water. The compounding ratio of the component (A) of hydrophilic nonionic surfactant and the component (C) of hydrophobic powder is component (A) / component (C) = 1/100 to 20/100 (wt%), preferably, component (A) / component (C) = 1/100 to 15/100 (wt%). If the amount of the component (A) of hydrophilic nonionic surfactant is less than the component (A) / component (C) = 1/100 (wt%), it cannot be mixed with water, and if the hydrophilic nonionic surfactant is more than the component (A) / component (C) = 20/100 (wt%), since the amount of the surfactant becomes excessive, the sense of use of the cosmetics is worsened and affect is provided to the stability of the cosmetics.

It is preferable that the component (A) of the hydrophilic nonionic surfactant, the component (B) of water, and the component (C) of hydrophobic powder are each contained in an amount of 0.1 wt% or more based on the total amount of the cosmetics. The preferable ratio of these three components is the compounding ratio (A) / (C) = 0.1 / 100 to 90/100 (wt%) of the hydrophilic nonionic surfactant of (A) and the hydrophobic powder of (C), preferably, (A) / (C) = 1/100 to 50/100 (wt%). If the amount of (A) hydrophilic nonionic surfactant is less than (A) / (C) = 1/100 (wt%), the affinity and dispersibility with water is worsened. If the amount of the hydrophilic nonionic surfactant is more than (A) / (C) = 90/100 (wt%), the amount of the surfactant becomes excessive, which worsens the usability of the cosmetic and affects the stability of the cosmetic. The compounding amount of the hydrophobic powder and the component (B) varies depending on the form of the cosmetic.

The method for producing cosmetics of the present invention is suitable for blending a hydrophobic powder with an aqueous cosmetic, but it can also be applied to an emulsified cosmetic, an oil-based cosmetic, and a powder-based cosmetic. The cosmetics referred to in the present invention include, as skin care cosmetics, emollient creams, cold creams, whitening creams, milky lotions, lotions, beauty liquids, packs, carmine lotions, liquid facial cleansers, facial cleansing foams, facial cleansing creams, facial cleansing powders, makeup cleansing, body gloss, sunscreen or sun cream or lotion, etc., as make-up cosmetics, make-up bases, powder foundations, liquid foundations, oil-based foundations, stick foundations, pressed powders, face powders, white powders, lipsticks, lipstick overs, lip gloss, concealer, blusher, eye shadow, eyebrow, eyeliner, mascara, water-based nail enamel, oil-based nail enamel, emulsified nail enamel, enamel top coat, enamel base coat, etc., as hair cosmetics, hair gloss, hair cream, hair shampoo, hair rinse, hair color, hair brushing agent, etc., as anti-sweat cosmetics, creams, lotions, powders, spray-type deodorant products, etc., as others, milky lotions, soaps, bathing agents, perfumes, etc.

### [Examples]

The present invention will be described in detail below with reference to Examples and Comparative Examples.

### (Examples 1 to 11, Comparative Examples 1 to 4)

A hydrophilic surfactant is added to aqueous phase having the composition shown in Table 1 below, the mixture is heated to 60°C, hydrophobic powder is gradually added under stirring with a disper mixer, and then dispersed for 15 minutes at a peripheral speed of 10 to 12 m/s. The state of dispersion of the hydrophobic powder in the aqueous phase was evaluated according to the following criteria.

### (Evaluation criteria)

○: Being dispersed in the aqueous phase to be a dispersion liquid that is uniform and flowable.
Δ: Being dispersed in the aqueous phase, but becomes a paste substance having whelk feeling and not-flowable.
×: Hydrophobic powder did not disperse in the aqueous phase and became a clay-like substance.

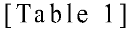

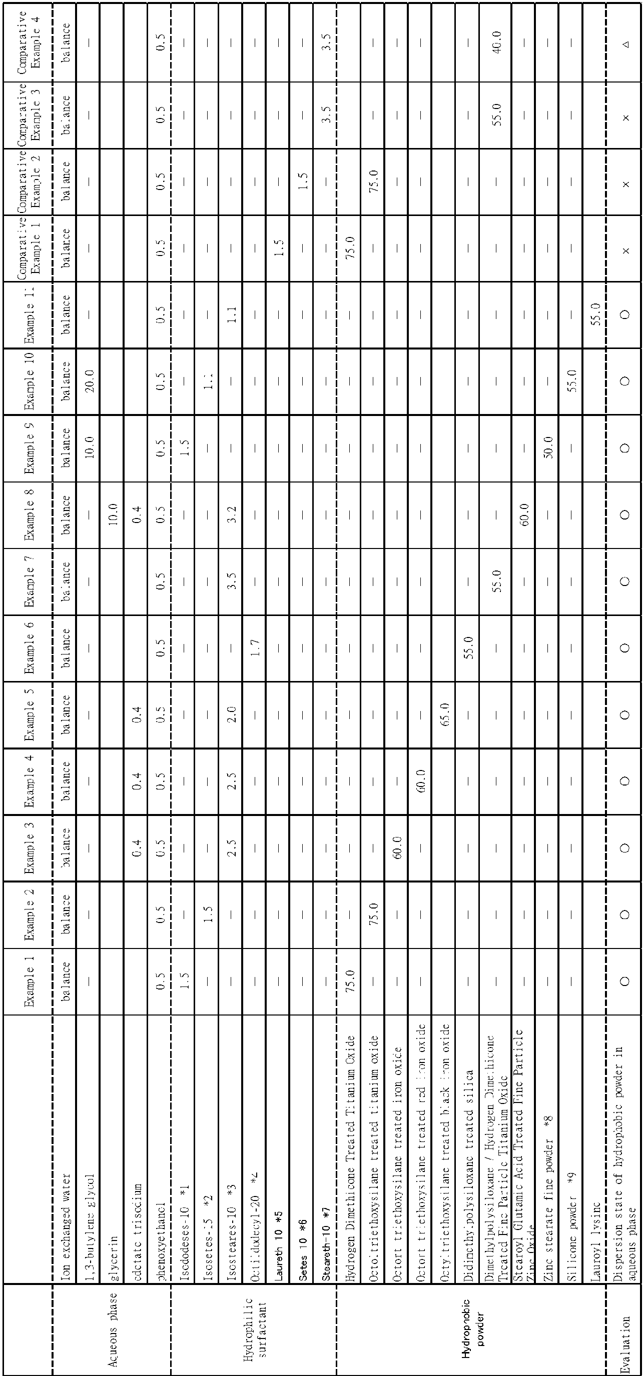

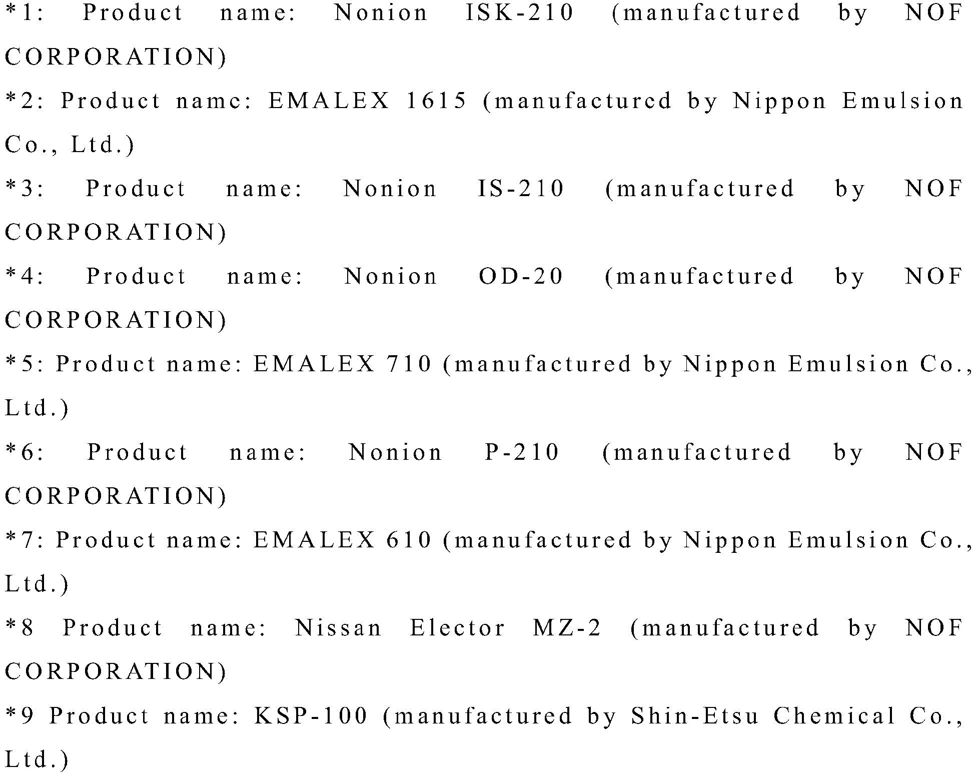

### (Discussion of evaluation results)

A process of the present invention of mixing the component (A) of hydrophilic nonionic surfactant as a monoether compound of a branched higher alcohol having 12 to 20 carbons and a polyoxyethylene group and the component (B) of water, and then mixing them with the component (C) of hydrophobic powder could provide dispersion liquids in which the hydrophobic powder flows uniformly. On the other hand, in Comparative Examples 1 to 3 in which the hydrophilic nonionic surfactant is used, all the hydrophobic powder could not be dispersed in water and became a clay-like substance on the way. In Comparative Example 4 in which the blending amount of the hydrophobic powder of Comparative Example 3 was reduced by about 27 wt%, produced was a non-fluid paste with a feeling of whelk and did not become a uniform fluid.

### (Example 12 Production of O / W type emulsified foundation)

An O / W type emulsified foundation having the composition shown in Table 2 was produced.

**[Table 2]**

| | Component | Example 12 |
|---|---|---|
| Oil layer component | Isohexadecane | 13.0(wt%) |
| | Glyceryl tri2-ethylhexanoate | 5.5 |
| | 2-ethylhexyl paramethoxycinnamate | 5.0 |
| | Behenyl alcohol | 1.0 |
| | Dibutylhydroxytoluene | 0.05 |
| Aqueous layer component | Dispersion liquid of Example 2 (titanium oxide) | 8.0 |
| | Dispersion liquid of Example 3 (yellow iron oxide) | 3.1 |
| | Dispersion liquid of Example 4 (red iron oxide) | 2.1 |
| | Dispersion liquid of Example 5 (black iron oxide) | 0.2 |
| | BG | 5.0 |
| | Ethanol | 5.0 |
| | Carbomer | 0.2 |
| | Triethanolamine | 0.1 |
| | Phenoxyethanol | 0.5 |
| | Ion-exchanged water | balance |

### (Production method)

A: The oil layer components were well dispersed and mixed.
B: The aqueous layer components were well dispersed and mixed.
C: A was added to B and emulsified with a homomixer to obtain an O / W type emulsified foundation.

The cosmetic of the present invention had a fresh and smooth feeling of use, had a uniform finish, and had an excellent makeup durability.

### (Example 13 Production of W / O type emulsified foundation)

A W / O type emulsified foundation having the composition shown in Table 3 was produced.

**[Table 3]**

| | Components | Example 13 |
|---|---|---|
| Oil layer component | Decamethylcyclopentasiloxane | 11.0(wt%) |
| | Isohexadecane | 5.5 |
| | Triethylhexanoin | 5.0 |
| | 2-ethylhexyl paramethoxycinnamate | 8.0 |
| | PEG-9 Polydimethylsiloxyethyl Dimethicone | 4.0 |
| | Silicone treated fine particle zinc oxide | 6.5 |
| Aqueous layer component | Dispersion liquid of Example 1 (titanium oxide) | 7.5 |
| | Dispersion liquid of Example 3 (yellow iron oxide) | 3.0 |
| | Dispersion liquid of Example 4 (red iron oxide) | 1.2 |
| | Dispersion liquid of Example 5 (black iron oxide) | 0.3 |
| | BG | 6.0 |
| | Phenoxyethanol | 0.8 |
| | Ion-exchanged water | to100.0 |

### (Production method)

A: The oil layer components were well dispersed and mixed.
B: The aqueous layer components were well dispersed and mixed.
C: B was added to A and emulsified with a homomixer to obtain a W / O type emulsified foundation.

The cosmetic of the present invention had a fresh and smooth feeling of use, had a uniform finish, and had an excellent makeup durability.

### (Example 14 Production of water-based sun-cut lotion)

An aqueous sun-cut lotion having the composition shown in Table 4 was produced.

**[Table 4]**

| | Components | Example 14 |
|---|---|---|
| Oil layer component | Decamethylcyclopentasiloxane | 15.0(wt%) |
| | Didimethylpolysiloxane (6cs) | 5.0 |
| | Triethylhexanoin | 6.0 |
| Aqueous layer component | Dispersion liquid of Example 7 (fine particle titanium oxide) | 8.0 |
| | Dispersion liquid of Example 8 (fine particle zinc oxide) | 10.0 |
| | PEG-10 dimethicicon | 3.0 |
| | Glyceryl monostearate | 1.5 |
| | BG | 5.0 |
| | Ethanol | 5.0 |
| | Purified water | balance |
| | in-vitro SPF value | 42.3 |

### (Production method)

A: The oil layer components were well dispersed and mixed.
B: The aqueous layer components were well dispersed and mixed.
C: A was added to B and emulsified with a homomixer to obtain an aqueous sun-cut lotion.

The cosmetic of the present invention had a fresh and smooth feeling of use, had a uniform finish, and had an excellent makeup durability.

### (Example 15 Production of O / W type sunscreen cosmetics)

An O / W type sunscreen cosmetic having the composition shown in Table 5 was produced.

**[Table 5]**

| | Components | Example 15 |
|---|---|---|
| Oil layer component | Isododecane | 8.0 |
| | Glyceryl octanate | 4.0 |
| | Didimethylpolysiloxane (10cs) | 3.0 |
| | Setostearyl alcohol | 1.0 |
| | 2-ethylhexyl paramethoxycinnamate | 5.0 |
| Aqueous layer component | Dispersion liquid of Example 8 (fine particle zinc oxide) | 12.0 |
| | PEG-80 hydrogenated castor oil | 1.0 |
| | Acrylic acid / acryloyldimethyltaurine Na copolymer | 0.2 |
| | Xanthan gum | 0.1 |
| | Phenoxyethanol | 0.7 |
| | Glycerin | 5.0 |
| | Ethanol | 5.0 |
| | Purified water | balance |
| | in-vitro SPF value | 37.2 |

### (Production method)

A: The oil layer components were well dispersed and mixed.
B: The aqueous layer components were well dispersed and mixed.
C: A was added to B and emulsified with a homomixer to obtain an O / W type sunscreen cosmetic.

The cosmetic of the present invention had a fresh and smooth feeling of use, had a uniform finish, and had an excellent makeup durability.

### (Example 16 Production of W / O type sunscreen cosmetics)

A W / O type sunscreen cosmetic having the composition shown in Table 6 was produced.

**[Table 6]**

| | Components | Example 16 |
|---|---|---|
| Oil layer component | Decamethylcyclopentasiloxane | 10.0(wt%) |
| | Isododecane | 9.0 |
| | Diisopropyl sebacate | 8.0 |
| | PEG-10 dimethicone | 4.0 |
| | Diethylaminohydroxybenzoyl hexyl benzoate | 8.0 |
| Aqueous layer component | Dispersion liquid of Example 7 (fine particle titanium oxide) | 5.0 |
| | Dispersion liquid of Example 8 (fine particle zinc oxide) | 10.0 |
| | BG | 10.0 |
| | Erythritol | 2.0 |
| | Phenoxyethanol | 0.5 |
| | Purified water | balance |
| | in-vitro SPF value | 47.5 |

### (Production method)

A: The oil layer components were well dispersed and mixed.
B: The aqueous layer components were well dispersed and mixed.
C: B was added to A and emulsified with a homomixer to obtain a W / O type sunscreen cosmetic.

The cosmetic of the present invention had a fresh and smooth feeling of use, had a uniform finish, and had an excellent makeup durability.

### (Example 17 Production of powder foundation)

A powder foundation having the composition shown in Table 7 was produced.

**[Table 7]**

| | Components | Example 17 |
|---|---|---|
| Powder component | Na lauroyl glutamate treated talc | balance (wt%) |
| | Na lauroyl glutamate treated sericite | 16.0 |
| | Na lauroyl glutamic acid treated mica | 10.0 |
| | Na lauroyl glutamate Na titanium oxide | 8.0 |
| | Na Lauroyl Glutamic Acid Treated Iron Oxide | 2.8 |
| | Na Lauroyl Glutamic Acid Treated Red Iron Oxide | 1.3 |
| | Na Lauroyl Glutamic Acid Treated Black Iron Oxide | 0.2 |
| | Dispersion liquid of Example 6 | 5.0 |
| | Dispersion liquid of Example 9 | 4.0 |
| Oily component | 2-ethylhexyl paramethoxycinnamate | 3.0 |
| | Glyceryl tri2-ethylhexanoate | 3.5 |
| | Didimethylpolysiloxane (20cs) | 2.0 |
| | Squalene | 2.0 |
| | Sorbitan sesquioleate | 0.5 |
| | Antibacterial agent | Appropriate amount |
| | Antioxidant | Appropriate amount |

### (Production method)

A: The powder components were well dispersed and mixed.
B: The oily components were well mixed and dissolved.
C: B was added to A, mixed and pulverized, and then molded into a gold plate through a screen to obtain a powder foundation.

The cosmetic of the present invention had a fresh and smooth feeling of use, had a uniform finish, and had an excellent makeup durability.

### (Example 18 Production of oily solid foundation)

An oily solid foundation having the composition shown in Table 8 was produced.

**[Table 8]**

| | Components | Example 18 |
|---|---|---|
| Oily component | Polyglyceryl-2 triisostearate | 8.5(wt%) |
| | Propylene glycol dicaprate | 6.0 |
| | Didimethylpolysiloxane (20cs) | 9.0 |
| | Di (phytosteryl / octyldodecyl) lauroyl glutamate | 6.0 |
| | Vaseline | 7.5 |
| | Polyethylene wax | 4.0 |
| | Candelilla wax | 1.5 |
| | 2-ethylhexyl paramethoxycinnamate | 3.0 |
| Powder component | Alkylsilane treated talc | balance |
| | Hydrophobic Titanium Oxide of Example 1 | 9.0 |
| | Hydrophobic Yellow iron oxide of Example 3 | 5.0 |
| | Hydrophobic Red iron oxide of Example 4 | 3.3 |
| | Hydrophobic Black iron oxide of Example 5 | 0.3 |
| | Preservative | Appropriate amount |

### (Preparation method of powder component)

The hydrophilic hydrophobic titanium oxide of Example 1 was prepared by putting the dispersion liquid of Example 1 in a stainless steel vat, drying at 80 °C. for 24 hours, and then pulverizing. Hydrophobic yellow iron oxide of Example 3, hydrophobic red iron oxide of Example 4, and hydrophobic black iron oxide of Example 5 were also prepared in the same manner.

### (Production method)

A: The powder components were well dispersed and mixed.
B: The oily components were well mixed and dissolved.
C: B was added to A, treated with a hot roller, poured into a gold plate, and cooled and molded to obtain an oily solid foundation.

The cosmetic of the present invention had a fresh and smooth feeling of use, had a uniform finish, and had an excellent makeup durability.

### (Example 19 Production of aqueous face powder)

An aqueous face powder having the composition shown in Table 9 was produced.

**[Table 9]**

| | Components | Example 19 |
|---|---|---|
| Powder component | Talc | 15.0(wt%) |
| | Synthetic mica | 3.0 |
| | Dispersion liquid of Example 1 (titanium oxide) | 10.0 |
| | Dispersion liquid of Example 9 (zinc stearate fine powder | 5.0 |
| | Dispersion liquid of Example 10 (silicone powder) | 7.5 |
| | Dispersion liquid of Example 11 (lauroyl lysine) | 10.0 |
| Aqueous layer component | BG | 5.0 |
| | Glycerin | 5.0 |
| | Ethanol | 5.0 |
| | EDTA • 2Na | 0.2 |
| | Phenoxyethanol | 0.3 |
| | SEPINOV P88 | 0.2 |
| | Ion-exchanged water | balance |

| | | |
|---|---|---|
| ^{∗} As (Na acrylate / acryloyl dimethyl taurine / dimethyl acrylamide) cross polymer, SEPINOV P88 from SEPPIC S.A was used. | | |

### (Production method)

A: The powder components were well mixed.
B: The aqueous layer components were mixed and dissolved.
C: A was added to the B and stirred well to obtain an aqueous face powder.

The cosmetic of the present invention had a fresh and smooth feeling of use, had a uniform finish, and had an excellent makeup durability.

### (Example 20: Production of aqueous eye shadow)

An aqueous eyeshadow having the composition shown in Table 10 was produced.

**[Table 10]**

| | Components | Example 20 |
|---|---|---|
| Powder component | Talc | 5.0(wt%) |
| | Pearl pigment | 25.0 |
| | Dispersion liquid of Example 3 (yellow iron oxide) | 1.4 |
| | Dispersion liquid of Example 4 (red iron oxide) | 0.6 |
| | Dispersion liquid of Example 5 (black iron oxide) | 0.2 |
| | Dispersion liquid of Example 11 (lauroyl lysine) | 9.0 |
| Aqueous layer component | BG | 5.0 |
| | Glycerin | 5.0 |
| | Ethanol | 5.0 |
| | EDTA • 2Na | 0.2 |
| | Citric acid | 0.03 |
| | Na citrate | 0.12 |
| | Phenoxyethanol | 0.3 |
| | Polyacrylate crosspolymer-6 | 0.2 |
| | Ion-exchanged water | balance |

| | | |
|---|---|---|
| ^{∗} As polyacrylate crosspolymer-6, is SEPIMAX ZEN from a SEPPIC S.A was used. | | |

### (Production method)

A: The powder components were well mixed.
B: The aqueous layer components were mixed and dissolved.
C: A was added to the B and stirred well to obtain an aqueous eye shadow.

The cosmetic of the present invention had a fresh and smooth feeling of use, had a uniform finish, and had an excellent makeup durability.

### (Example 21: Production of lipstick)

A lipstick having the compositions shown in Table 11 were produced.

**[Table 11]**

| | Components | Example 21 |
|---|---|---|
| Oil layer component | Dextrin palmitate / ethylcaproate | 8..0(wt%) |
| | Cetyl octanate | 20.0 |
| | PEG-10 dimethicone | 3.0 |
| | Decamethylcyclopentasiloxane | 40.0 |
| Powder component | Bentonite | 0.8 |
| | Dispersion liquid of Example 1 (titanium oxide) | 5.0 |
| | Dispersion liquid of Example 4 (red iron oxide) | 1.2 |
| Aqueous layer component | BG | 5.0 |
| | Sodium chloride | 0.5 |
| | Purified water | balance |

### (Production method)

A: The oil layer components were well mixed.
B: The powder component was mixed with the component A and dispersed by a roller.
C: B was added to A and mixed uniformly.
D: The aqueous layer components were mixed and heated.
E: D was added to C and emulsified to obtain lipstick.

The cosmetic of the present invention had a fresh and smooth feeling of use, had a uniform finish, and had an excellent makeup durability.

### (Example 22 Production of antiperspirant)

An antiperspirant having the composition shown in Table 12 was produced.

**[Table 12]**

| | Components | Example 22 |
|---|---|---|
| Powder component | Dispersion liquid of Example 8 (fine particle zinc oxide) | 2.0(wt%) |
| | Dispersion liquid of Example 10 (silicone powder) | 5.5 |
| | Dispersion liquid of Example 11 (lauroyl lysine) | 9.0 |
| Aqueous layer component | Sodium chloride | 0.1 |
| | Ethanol | 25.0 |
| | BG | 2.0 |
| | Polyoxyethylene sorbitan monolaurate | 0.2 |
| | Phenoxyethanol | 0.3 |
| | Ion-exchanged water | balance |

### (Production method)

A: The powder components were well mixed.
B: The aqueous layer components were mixed and dissolved.
C: A was added to B and mixed to obtain an antiperspirant.

The cosmetic of the present invention had a fresh feeling of use, was not sticky, and was excellent in comfort.

### (Example 23: Production of treatment)

A treatment having the composition shown in Table 13 were produced by the following methods.

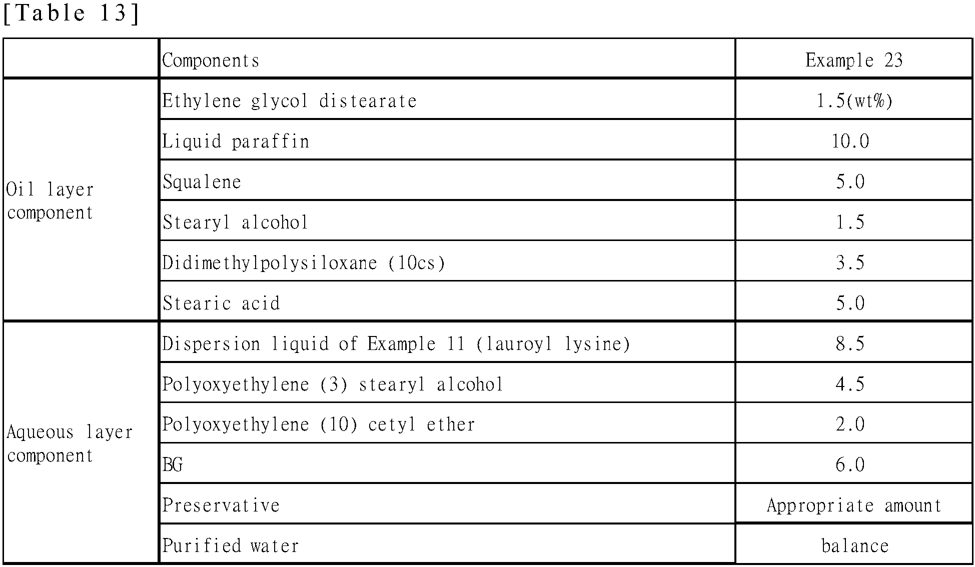

### (Production method)

A: The oil layer components were heated and mixed.
B: Aqueous layer components were dispersed and mixed.
C: B was added to A and mixed well to obtain a treatment.

The cosmetic of the present invention had a fresh and smooth feeling of use, had a uniform finish, and had an excellent makeup durability. The cosmetic of the present invention had a fresh feeling of use, provided easiness of combing, and provided excellent smoothness of hair.

### (Examples 24 to 32, Comparative Examples 5 to 11)

After adding a hydrophilic surfactant to the aqueous phase having the composition shown in Table 14 below and heating to 60 °C ., the hydrophobic powder is gradually added under stirring with a disper mixer, and then dispersed for 15 minutes at a peripheral speed of 10 m/s to obtain a dispersion liquid. The state of dispersion of the hydrophobic powder in the aqueous phase was evaluated according to the following criteria. As to Example 29 and Comparative Example 10, a hydrophobic powder and a hydrophilic surfactant were mixed to prevent scattering as a mixture, and hen the aqueous phase was added to the mixture under stirring with a disper mixer, and the same operation was performed to obtain a dispersion liquid.

### (Evaluation criteria)

○: Being dispersed in the aqueous phase to be a dispersion liquid that is uniform and flowable.
Δ: Being dispersed in the aqueous phase, but becomes a paste substance having whelk feeling and not-flowable.
×: All of hydrophobic powder did not disperse in the aqueous phase and became a clay-like substance.

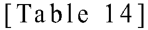

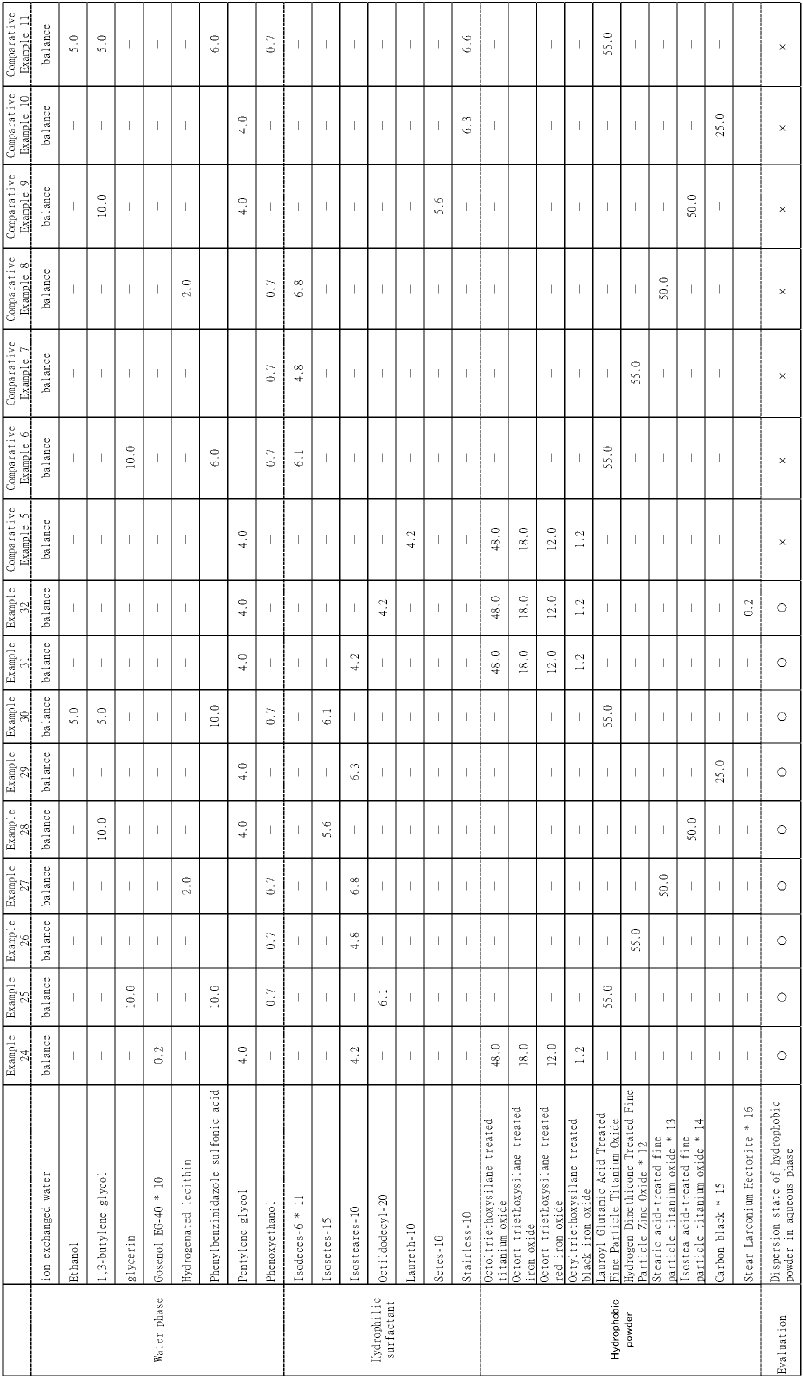

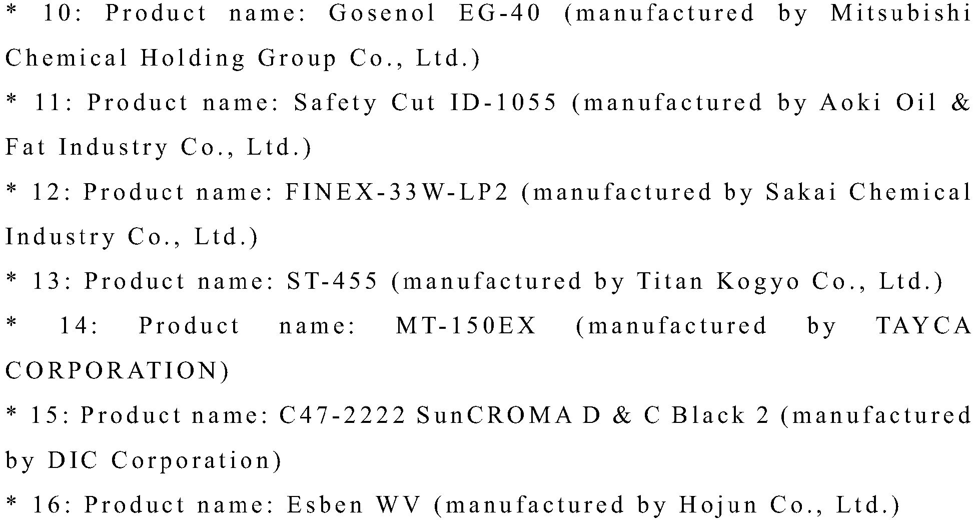

### (Discussion of evaluation results)

A process of the present invention of mixing the component (A) of hydrophilic nonionic surfactant as a monoether compound of a branched higher alcohol having 12 to 20 carbons and a polyoxyethylene group and the component (B) of water, and then mixing them with the component (C) of hydrophobic powder could provide dispersion liquids in which the hydrophobic powder flows uniformly. On the other hand, in Comparative Examples 5 to 11 in which the hydrophilic nonionic surfactant is used, all the hydrophobic powder could not be dispersed in water and became a clay-like substance on the way. Although Example 29 is a method in which a hydrophilic surfactant is mixed with a hydrophobic powder and then the mixture is added to the aqueous phase, a uniform dispersion liquid could be prepared. All of the comparative examples were non-fluid pastes and did not become uniform dispersion liquids.

### (Example 33 Production of a pouring type powder foundation)

A pouring type powder foundation having the composition shown in Table 15 was produced by the following method.

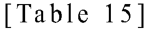

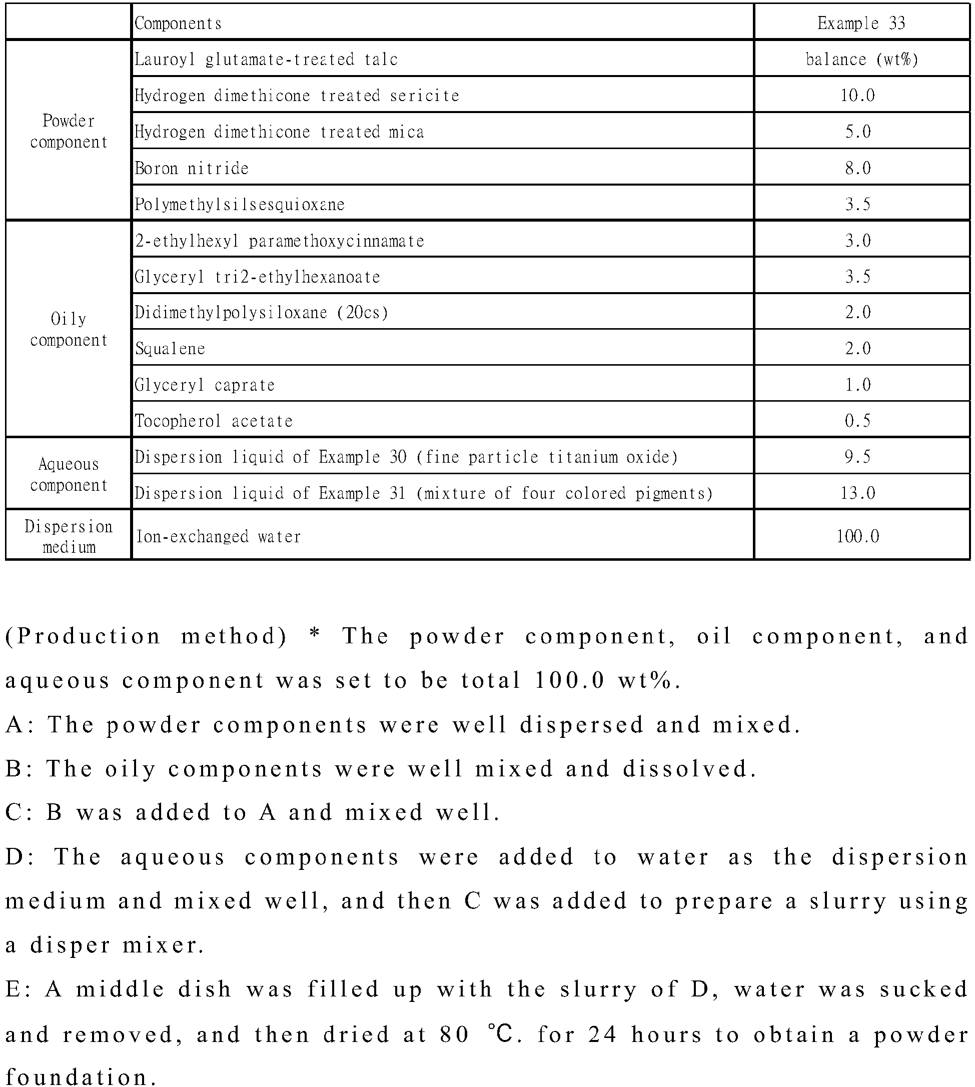

The cosmetic of the present invention had a fresh and smooth feeling of use, had a uniform finish, and had an excellent makeup durability. In addition, it had an excellent impact resistance.

### (Example 34 Production of aqueous liquid eyeliner)

An aqueous liquid eyeliner having the composition shown in Table 16 was produced by the following method.

**[Table 16]**

| | Components | Example 34 |
|---|---|---|
| Aqueous layer component | Alkyl acrylate copolymer liquid (solid content concentration: 33 wt% ) | 9 .0(wt%) |
| | Urethane acrylic composite resin emulsion (solid content concentration | 7.0 |
| | Diethylhexyl sulfosuccinate Na | 0.5 |
| | Dispersion liquid of Example 29 (carbon black) | 32.0 |
| | BG | 8.0 |
| | Ethanol | 4.0 |
| | Preservative | Appropriate amount |
| | Ion-exchanged water | balance |

### (Production method)

A: The aqueous phase components were well mixed with a disper mixer to obtain an aqueous liquid eyeliner.

The cosmetic of the present invention had a fresh feeling and a smooth feeling of use, and was excellent in a uniform finish.

### (Example 35 and Example 36 Production of W / O type sunscreen cosmetics)

W / O type sunscreen cosmetics having the composition shown in Table 17 were produced by the following method.

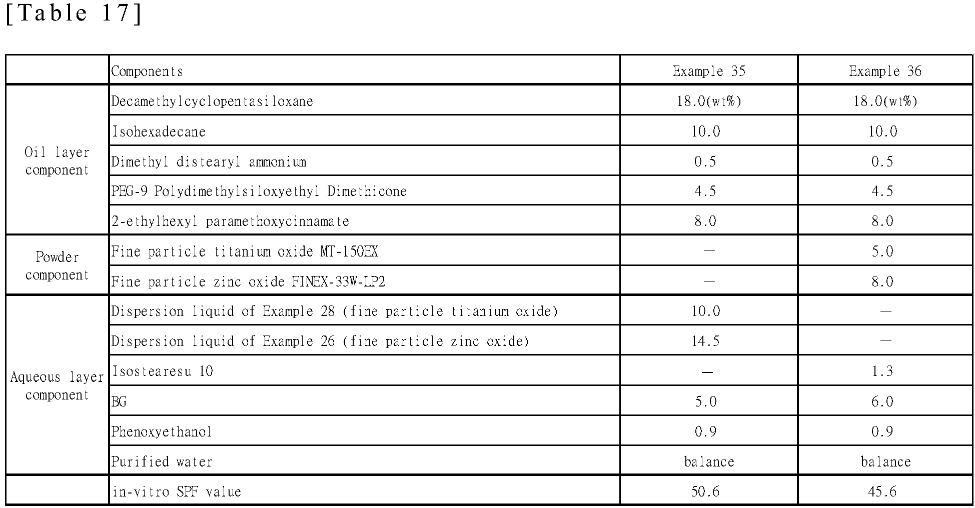

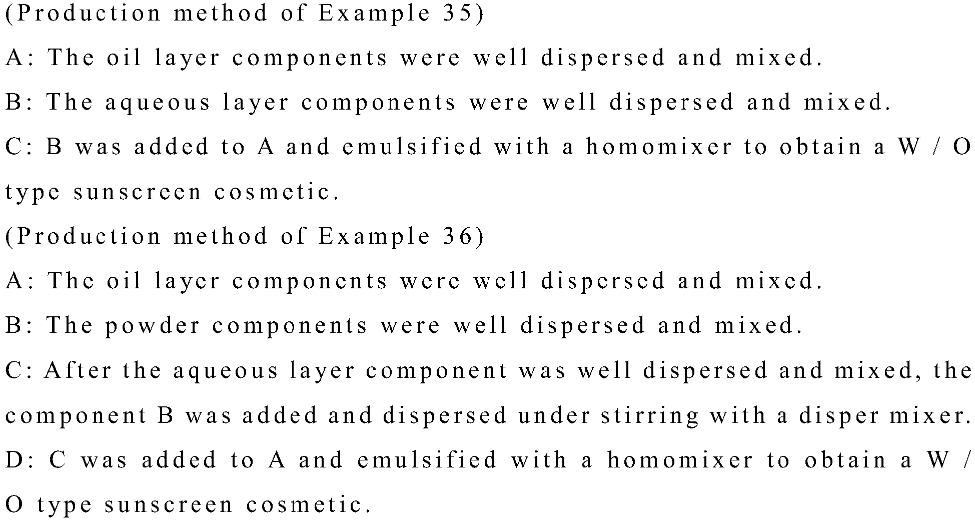

The cosmetic of the present invention had a fresh and smooth feeling of use, had a uniform finish, and had an excellent makeup durability.

### (Example 37 and Example 38 Production of O / W type sunscreen cosmetics)

O / W type sunscreen cosmetics having the composition shown in Table 18 were produced by the following method.

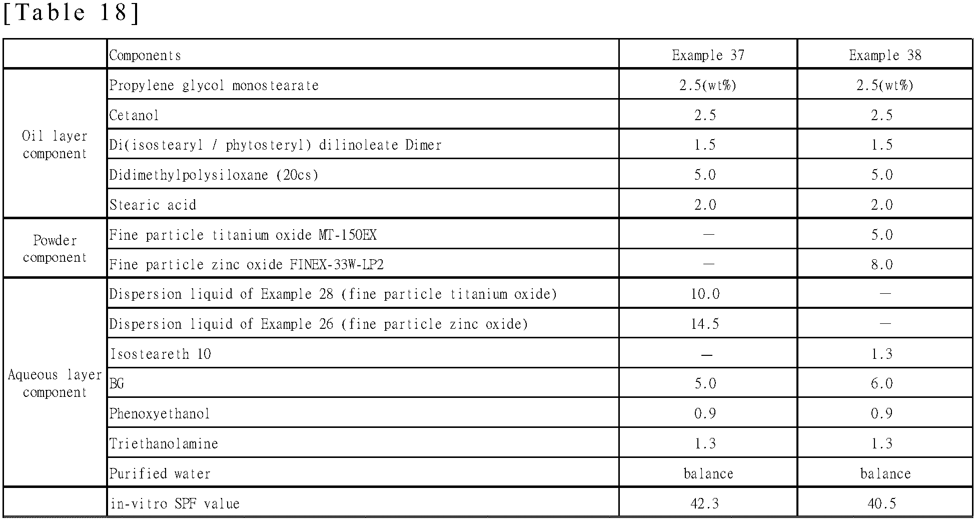

The cosmetic of the present invention had a fresh and smooth feeling of use, had a uniform finish, and had an excellent makeup durability.

The present invention also includes the following preferred forms.

### (Form 1)

A producing method of cosmetic containing, respectively, 1 wt% or more of water and hydrophobic powder, wherein
the method comprises a step of mixing (A) hydrophilic nonionic surfactant as monoether compound of branched higher alcohol having 12 to 20 carbons and polyoxyethylene group and (B) water, subsequently mixing with (C) hydrophobic powder.

### (Form 2)

The producing method of cosmetic according to Form 1, wherein (B) water contains polyol at less than 50 wt%.

### (Form 3)

The producing method of cosmetic according to Form 1 or 2, wherein (C) is organic powder.

### (Form 4)

The producing method of cosmetic according to Form 1 or 2, wherein (C) is hydrophobic inorganic powder.

### (Form 5)

The producing method of cosmetic according to any one of Forms 1 to 4, wherein (A) is a monoether compound of branched higher alcohol having 16 to 20 carbons and polyoxyethylene group.

### (Form 6)

A cosmetic obtained by the producing method according to Forms 1 to 5.

In addition, the present invention is also described as the following modes.

### (Mode 1)

A producing method of cosmetic containing, respectively, at least 0.1 wt% or more of the following components
(A) hydrophilic nonionic surfactant as monoether compound of branched higher alcohol having 14 to 20 carbons and 5 to 20 moles of ethylene oxide;
(B) water;
(C) hydrophobic powder;
wherein the method comprises a step of mixing the component (A) and the component (B), and subsequently mixing and dispersing the component (C).

### (Mode 2)

A producing method of cosmetic containing, respectively, 0.1 wt% or more of at least the following components
(A) hydrophilic nonionic surfactant as monoether compound of branched higher alcohol having 14 to 20 carbons and 5 to 20 moles of ethylene oxide;
(B) water;
(C) hydrophobic powder;
wherein the method comprises a step of mixing or contacting the component (A) and the component (C), and subsequently adding the resultant mixture to the component (B) followed by mixing and dispersing.

### (Mode 3)

The producing method of cosmetic according to Mode 1 or 2, wherein the component (B) contains 10 wt% or less of a component capable of being mixed or solubilized in the component (B), that is other than ethyl alcohol or polyhydric alcohol.

### (Mode 4)

The producing method of cosmetic according to Mode 1 or 2, wherein the component (B) contains 50 wt% or less of polyhydric alcohol.

### (Mode 5)

The producing method of cosmetic according to Mode 1 or 2, wherein the component (B) contains 10 wt% or less of a component capable of being mixed or solubilized in the component (B) other than ethyl alcohol or polyhydric alcohol, and further contains 50 wt% or less of polyhydric alcohol.

### (Mode 6)

The producing method of cosmetic according to any one of Modes 1 to 5, wherein the component (C) of hydrophobic powder is organic powder.

### (Mode 7)

The producing method of cosmetic according to any one of Modes 1 to 5, wherein the component (C) of hydrophobic powder is inorganic powder which had been subjected to an organic treatment.

### (Mode 8)

The producing method of cosmetic according to any one of Modes 1 to 7, wherein the component (A) is monoether compound of branched higher alcohol having 16 to 20 carbons and 10 to 20 moles of ethylene oxide.

### (Mode 9)

A cosmetic obtained by the producing method according to any one of Modes 1 to 8.

## Claims

1. A producing method of cosmetic containing, respectively, 0.1 wt% or more of at least the following components:
(A) hydrophilic nonionic surfactant as monoether compound of branched higher alcohol having 14 to 20 carbons and 5 to 20 moles of ethylene oxide;
(B) water;
(C) hydrophobic powder,
wherein the method comprises a step of mixing the component (A) and the component (B), and subsequently mixing and dispersing the component (C).

2. A producing method of cosmetic containing, respectively, 0.1 wt% or more of at least the following components:
(A) hydrophilic nonionic surfactant as monoether compound of branched higher alcohol having 14 to 20 carbons and 5 to 20 moles of ethylene oxide;
(B) water;
(C) hydrophobic powder,
wherein the method comprises a step of mixing or contacting the component (A) and the component (C), and subsequently adding the resultant mixture to the component (B) followed by mixing and dispersing.

3. The producing method of cosmetic according to Claim 1 or 2, wherein the component (B) contains 10 wt% or less of a component capable of being mixed or solubilized in the component (B), that is other than ethyl alcohol or polyhydric alcohol.

4. The producing method of cosmetic according to Claim 1 or 2, wherein the component (B) contains 50 wt% or less of polyhydric alcohol.

5. The producing method of cosmetic according to Claim 1 or 2, wherein the component (B) contains 10 wt% or less of a component capable of being mixed or solubilized in the component (B), that is other than ethyl alcohol or polyhydric alcohol, and further contains 50 wt% or less of polyhydric alcohol.

6. The producing method of cosmetic according to any one of Claims 1 to 5, wherein the component (C) of hydrophobic powder is organic powder.

7. The producing method of cosmetic according to any one of Claims 1 to 5, wherein the component (C) of hydrophobic powder is inorganic powder which had been subjected to an organic treatment.

8. The producing method of cosmetic according to any one of Claims 1 to 7, wherein the component (A) is monoether compound of branched higher alcohol having 16 to 20 carbons and 10 to 20 moles of ethylene oxide.

9. A cosmetic obtained by the producing method according to any one of Claims 1 to 8.

## Amended claims

Statement under Art. 19.1 PCT
1. (Cancelled)

2. (Cancelled)

3. (Cancelled)

4. (Cancelled)

5. (Cancelled)

6. (Cancelled)

7. (Cancelled)

8. (Cancelled)

9. (Cancelled)

10. (New) A producing method of cosmetic containing, respectively, 0.1 wt% or more of the following components:
(A) hydrophilic nonionic surfactant as monoether compound of a branched higher alcoholhhaving 14 to 20 carbons and 5 to 20 moles of ethylene oxide;
(B) water; and
(C) hydrophobic powder,
   wherein the method comprises
   a step of preparing an aqueous dispersion liquid by mixing and dispersing the components (A), (B) and (C), and
   a step of preparing a cosmetic component by mixing the aqueous dispersion liquid and oil layer components.

11. (New) The producing method of cosmetic according to Claim 10, wherein, in the step of preparing the aqueous dispersion liquid, the components (A) and the component (B) are mixed, and subsequently mixed and dispersed with the component (C).

12. (New) The producing method of cosmetic according to Claim 10, wherein, in the step of preparing the aqueous dispersion liquid, the components (A) and the component (C) are mixed or contacted, and subsequently added to the component (B) followed by mixing and dispersing.

13. (New) The producing method of cosmetic according to any one of Claims 10 to 12, wherein
the method further comprises a step of preparing a powder component by mixing the aqueous dispersion liquid and the powder, and
in the step of preparing the powder component, the powder component and the oil layer components are mixed to prepare the cosmetic component.

14. (New) The producing method of cosmetic according to any one of Claims 10 to 13, wherein the component (B) contains 10 wt% or less of a component capable of being mixed or solubilized in the component (B), that is other than ethyl alcohol or polyhydric alcohol.

15. (New) The producing method of cosmetic according to any one of Claims 10 to 13, wherein the component (B) contains 50 wt% or less of polyhydric alcohol.

16. (New) The producing method of cosmetic according to any one of Claims 10 to 13, wherein the component (B) contains 10 wt% or less of a component capable of being mixed or solubilized in the component (B), that is other than ethyl alcohol or polyhydric alcohol, and further contains 50 wt% or less of polyhydric alcohol.

17. (New) The producing method of cosmetic according to any one of Claims 10 to 16, wherein the component (C) of hydrophobic powder is organic powder.

18. (New) The producing method of cosmetic according to any one of Claims 10 to 16, wherein the component (C) of hydrophobic powder is inorganic powder which had been subjected to an organic treatment.

19. (New) The producing method of cosmetic according to any one of Claims 10 to 18, wherein the component (A) is monoether compound of branched higher alcohol having 16 to 20 carbons and 10 to 20 moles of ethylene oxide.

20. (New) A cosmetic obtained by the producing method according to any one of Claims 10 to 19.
